# EUROPEAN PATENT APPLICATION

(11) **EP 2 280 079 A1**
(43) Date of publication of application: **02.02.2011**
(21) Application number: 09167011.7
(22) Date of filing: 31.07.2009
(51) Int. Cl.: C12Q 1/68

(54) **Ligation-based method of normalized quantification of nucleic acids**

(71) Applicant: Qiagen GmbH, 40724 Hilden (DE)
(72) Inventor: Löffert, Dirk, 40589 Düsseldorf (DE); Korfhage, Christian, 40764 Langenfeld (DE); Engel, Holger, 40724 Hilden (DE)
(74) Representative: Vossius & Partner

(57) **Abstract**

The present invention is related to normalized quantification of nucleic acids and to the normalization of quantities of nucleic acids in samples, e.g. mixtures of nucleic acids. The present invention relates to method for the normalization of the quantity of a nucleic acid to be quantifies in a sample to the total quantity of nucleic acid in the sample; or to the total quantity of a specific class of nucleic acid in the sample.

SEQUENCE LISTING

<110> Qiagen GmbH

<120> Ligation-based method of normalized quantification of nucleic
acids

<130> N2888 EP BLN

<160> 1

<170> PatentIn version 3.5

<210> 1
<211> 118
<212> DNA
<213> Artificial Sequence

<220>
<223> Open Circle Probe

<400> 1
tttttttttt tgcacaagct atggaacacc acgtaagaca taaaacggcc acataacttg 60

gctttaatgg acctccaatt ttgagtgtgg tgccatgtaa ggatgaatgt tttttttt 118

## Description

### Technical field of the invention

The present invention is in the field of Biology and Chemistry. In particular, the invention is in the field of Molecular Biology. More particular, the invention is in the field of quantification of nucleic acids and real-time PCR. Furthermore, the invention is related to normalize quantification of nucleic acids and to the normalization of quantities of nucleic acids in samples, *e.g*. mixtures of nucleic acids.

### Background of the invention

The quantification (quantitation) of specific nucleic acids in mixtures of nucleic acids is of importance in a number of applications in molecular biology, such as gene expression analysis or during purification of specific nucleic acids from a mixture of nucleic acids. In quantification methods, the concentrations and/or the relative or absolute amounts of specific nucleic acids in samples are determined. In particular, for the analysis of gene expression, for example for measuring mRNA levels in biological samples, a reproducible and comparative method is desired. For example, it is not always possible to obtain biological samples with comparable volume, amount of nucleic acid, cellular material or the like. Different samples can, for example, comprise RNA or DNA derived from different tissues, from different organisms or individuals, or cell culture samples that have been treated with different compounds.

In addition, sensitivity and selectivity of detection and quantification of nucleic acids in biological samples is of importance. For better comparison of the quantities of specific nucleic acids in two or more different (biological) samples or the comparison of the quantities of two or more different specific nucleic acids in a sample, a normalization of the quantities of the specific nucleic acids to the input nucleic acids or a specific class of input nucleic acid has to be performed. Quantities of specific nucleic acids can, *e.g*., be normalized by relating these quantities to an internal standard of the sample or to the overall, *i.e.* total, amount of nucleic acid or to the amount of a specific class of nucleic acid in the sample.

For conventional quantification of nucleic acids in (biological) samples, quantitative (real-time) PCR (qPCR) is widely used. For RNA, particularly mRNA., quantitative real-time reverse transcription PCR (RT-qPCR) is used in this field. Different approaches for the normalization of data obtained from quantitative PCR methods have been employed. Amang them is the normalization of the quantities of specific mRNAs to the quantities of one or more mRNAs of different reference genes, *e.g*. housekeeping or maintenance genes, such as beta actin, glyceraldehyde-3-phosphate dehydrogenase (GAPDH), hypoxanthine-guanine phosphoribosyl transferase (HPRT), or 28AS or 18S ribosomal RNA. However, the expression levels of such normalizer genes have been shown to vary depending on experimental conditions, preparation and source (*e.g*. tissue or cell type) of the samples and therefore they are not reliably indicative for the input nucleic acids. It is therefore commonly required to test a range of different housekeeping genes in a laborious and error-prone procedure in order to identify those which do not change between samples under investigation.

Other approaches, for example, rely on the normalization to the total content of DNA and/or RNA or the total content of *e.g*. ribosomal RNA (rRNA). As the content of ribosomal RNA in biological cells and samples is also subject to variations depending on a variety of factors, normalization to rRNA is also less preferred. Methods relying on the normalization to *e.g*. total nucleic acid content, total RNA content or total content of genomic DNA are also limited, *e.g.* by variations in these contents or the quality of the nucleic acid samples as well as the cumbersome need to accurately measure the amount of nucleic acid in each sample. Normalization to alien or artificial molecules, *e.g. in vitro* transcripts, that have been incorporated into a sample (*e.g*. a cell extract or a sample derived from a tissue) is also not in all cases an adequate procedure, since they do not represent the nucleic acid (*e.g*. genomic DNA, RNA, mRNA) content in a cell, *e.g.* with regard to quantity and integrity.

Besides, for comparison of normalized data and reproducibility of the experimental procedures, thorough documentation of the applied experimental conditions is required. This is particularly relevant, when the quantities of the nucleic acid of interest and the normalize nucleic acid are determined separately or using different methods.

Therefore, the technical problem underlying the present invention was to develop and to provide an improved, in particular a less laborious and error-prone, method for the normalization of quantities of nucleic acids that can be universally applied to biological samples.

### Summary of the invention

The present invention provides (a) robust and improved method(s) for the normalization of the quantity of a (specific) nucleic acid (*i.e*. a "target nucleic acid") in a sample or in a plurality of samples to the total quantity of nucleic acid in the sample(s); or the total quantity of a specific class of nucleic acid in the sample(s). The present invention also relates to a kit for the normalization of the quantity of a (specific) nucleic acid (*i.e.* a "target nucleic acid") in a sample or in a plurality of samples to the total quantity of nucleic acid in the sample(s); or the total quantity of a specific class of nucleic acid in the sample(s).

In the context of the present invention, a specific class of nucleic acid may be, *inter alia,* RNA, DNA, cDNA (complementary DNA), LNA (locked nucleic acid), mRNA (messenger RNA), mtRNA (mitochondrial), rRNA (ribosomal RNA), tRNA (transfer RNA), nRNA (nuclear RNA), siRNA (short interfering RNA), snRNA (small nuclear RNA), snoRNA (small nucleolar RNA), scaRNA (Small Cajal Body specific RNA), microRNA, dsRNA (doubled-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or any other class or subclass of nucleic acid which is distinguishable from the bulk nucleic acid in a sample.

The means and methods of the present invention comprise the use of nucleic acid probes. A nucleic acid probe according to the present invention is an oligonucleotide, nucleic acid or a fragment thereof, which is substantially complementary to a specific nucleic acid sequence.

In general, the present invention relates to a method for the normalization of the quantity of a nucleic acid to be quantified in a sample to (i) the total quantity of nucleic acid in the sample; or to (ii) the total quantity of a specific class of nucleic acid in the sample, comprising the steps of (a) providing a sample containing a nucleic acid to be quantified; (b) adding one or more first nucleic acid probes to the sample under conditions allowing for hybridization of at least a terminal region of said first nucleic acid probe to a specific binding site of nucleic acid in the sample such that double-stranded nucleic acids are created, wherein the first nucleic acid probe comprises one or more primer binding sites and optionally a probe binding site; (c) contacting the hybridized nucleic acids in the sample with a ligase under conditions that allow for ligation between any two terminal regions of such nucleic acid probe(s) whose 3' and 5' ends after hybridization are positioned in a way that ligation may occur; (d) quantifying the total amount of nucleic acid or the total amount of the specific class of nucleic acid by quantification of the ligation product from step (c) using two primers and optionally a second probe which is substantially complementary to a defined region of said first nucleic acid probe; (e) quantifying the nucleic acid to be quantified using a third probe substantially complementary to a region of said nucleic acid to be quantified; and (f) normalizing the quantity of the nucleic acid to be quantified by determining the ratio of the quantity of the nucleic acid to be quantified to the total quantity of nucleic acid or the total quantity of the specific class of nucleic acid.

The present invention also relates to a kit for the normalized quantification of nucleic acids in a sample, wherein the kit comprises: (a) one or more first nucleic acid probes substantially complementary to defined regions of nucleic acid or a specific class of nucleic acids in the sample, wherein the first nucleic acid probe comprises one or more primer binding sites and optionally a probe binding site; (b) a ligase; and (c) a second nucleic acid probe substantially complementary to said probe binding site on said first nucleic acid probe.

### Detailed description of the invention

The present invention provides a method for the normalization of the quantity of a nucleic acid to be quantified in a sample to (i) the total quantity of nucleic acid in the sample; or to (ii) the total quantity of a specific class of nucleic acid in the sample, comprising the steps of (a) providing a sample containing a nucleic acid to be quantified; (b) adding one or more first nucleic acid probes to the sample under conditions allowing for hybridization of at least a terminal region of said first nucleic acid probe to a specific binding site of nucleic acid in the sample such that double-stranded nucleic acids are created, wherein the first nucleic acid probe comprises one or more primer binding sites and optionally a probe binding site; (c) contacting the hybridized nucleic acids in the sample with a ligase under conditions that allow for ligation between any two terminal regions of such nucleic acid probe(s) whose 3' and 5' ends after hybridization are positioned in a way that ligation may occur; (d) quantifying the total amount of nucleic acid or the total amount of the specific class of nucleic acid by quantification of the ligation product from step (c) using primers and optionally a second probe which is substantially complementary to a defined region of said first nucleic acid probe; quantifying the nucleic acid to be quantified using a third probe substantially complementary to a region of said nucleic acid to be quantified.; and (f) normalizing the quantity of the nucleic acid to be quantified by determining the ratio of the quantity of the nucleic acid to be quantified to the total quantity of nucleic acid or the total quantity of the specific class of nucleic acid.

The primer and/or probe binding site on the first nucleic acid probe are arranged in such a way that amplification and/or detection or quantification of the first nucleic acid probe using primers and optionally a second nucleic acid probe may only occur after successful ligation in step (c).

A sample contains at least nucleic acid molecules comprising the nucleic acid to be quantified. The nucleic acids can be embedded in cells or organisms but can also be present in a cell free system. A sample may be a fluid, a lysate, solid matrix or anything else that contains nucleic acid molecules.

A nucleic acid in the context of the present invention relates to desoxyribo nucleic acid (DNA), ribo nucleic acid (RNA) or peptide nucleic acid (PNA). DNA and RNA are naturally occurring in organisms, however, they may also exist outside living organisms or may be added to organisms. The nucleic acid may be of any origin, *e.g*. viral, bacterial, archae-bacterial, fungal, ribosomal, eukaryotic or prokaryotic. It may be nucleic acid from any biological sample and any organism, tissue, cell or sub-cellular compartment. It may *e.g*. be nucleic acid from a plant, a fungus, an animal, and particularly human nucleic acid. The nucleic acid may be pre-treated before quantification, *e.g*. by isolation, purification or modification. Also artificial nucleic acid may be quantified. The length of the nucleic acids may vary. The nucleic acids may be modified, *e.g*. may comprise one or more modified nucleobases or modified sugar moieties (*e.g*. comprising methoxy groups). The backbone of the nucleic acid may comprise one or more peptide bonds as in peptide nucleic acid (PNA). The nucleic acid may comprise base analoga such as non-purine or non-pyrimidine analoga or nucleotide analoga. It may also comprise additional attachments such as proteins, peptides and/or or amino acids.

In particular embodiments of the invention the nucleic acid to be quantified is a RNA and the specific class of nucleic acid is RNA.

More in particular, the RNA to be quantified is RNA selected from the group consisting of mRNA, rRNA, TRNA, nRNA, siRNA, snRNA, snoRNA, scaRNA, microRNA, dsRNA, ribozyme, riboswitch and viral RNA and wherein the total quantity of RNA is selected from the group consisting of the total quantity of RNA, the total quantity of mRNA, the total quantity of rRNA, the total quantity of tRNA, the total quantity of nRNA, the total quantity of siRNA, the total quantity of snRNA, the total quantity of snoRNA, the total quantity of scaRNA, the total quantity of microFZNA, the total quantity of dsRNA, the total quantity of ribozyme, the total quantity of riboswitch, the total quantity of viral RNA.

In preferred embodiments of the invention the RNA to be quantified is a mRNA and the specific class of nucleic acid is mRNA.

Preferably, the specific binding site is specific for RNA or is a homopolymeric nucleotide sequence, *e.g*. a poly-A sequence or a poly-A tail. The poly-A tail can be as naturally occurring in the sample RNA. In case the RNA does not have a poly-A tail, experimental procedures can be employed to add a suitable homopolymeric tail to the 3' end of the RNA. Such tail can be composed of A, C ,G, or U bases or suitable base analogues. Addition of such tail can be performed chemically or enzymatically. Enzymes can be selected from the classes of poly-A polymerase, terminal transferase, ligase, or other suitable enzyme catalyzing addition or linkage of nucleotides to the 3' end of a nucleic acid.

In another embodiment of the invention the nucleic acid to be quantified is a DNA and the specific class of nucleic acid is DNA.

In particular embodiments of the invention the DNA to be quantified is DNA selected from the group consisting of cDNA, dsDNA, ssDNA, plasmid DNA, cosmid DNA, chromosomal DNA, viral DNA and mtDNA and wherein the total quantity of DNA is selected from the group consisting of the total quantity of DNA, the total quantity of cDNA, the total quantity of dsDNA, the total quantity of ssDNA, the total quantity of plasmid DNA, the total quantity of cosmid DNA, the total quantity of chromosomal DNA, the total quantity of viral DNA and the total quantity of mtDNA.

In preferred embodiments of the invention the DNA to be quantified is a cDNA and the specific class of nucleic acid is cDNA. Preferably, the specific binding site is a homopolymeric sequence, *e.g*. a poly-T sequence or a paly-T tail.

In some embodiments of the invention the first nucleic acid probe is DNA or RNA or an artificial or modified nucleic acid, *e.g*. peptide nucleic acid (PNA) or locked nucleic acid (LNA). Preferably the first nucleic acid probe is a DNA or RNA, most preferably a DNA.

The second nucleic acid probe may be DNA or RNA or an artificial or modified nucleic acid, *e.g*. peptide nucleic acid (PNA) or locked nucleic acid (LNA). The second nucleic acid probe has a sequence substantially complementary to the sequence of the probe binding site on said first nucleic acid probe. Therefore, the second nucleic acid probe is able to hybridize under certain conditions (temperature, salt concentration) known to a skilled person to the first nucleic acid probe. Hybridization of the second nucleic acid probe to the first nucleic acid probe may be indicative for a successful ligation of the first nucleic acid probe.

The third nucleic acid probe may be DNA or RNA or an artificial or modified nucleic acid, *e.g*. peptide nucleic acid (PNA) or locked nucleic acid (LNA). The third nucleic acid probe has a sequence substantially complementary to a sequence of the nucleic acid to be quantified. Therefore, the third nucleic acid probe is able to hybridize under certain conditions (temperature, salt concentration) known to a skilled person to the nucleic acid to be quantified.

In particular embodiments of the invention the quantifying steps comprise a method selected from the group consisting of gel electrophoresis, capillary electrophoresis, labelling reactions with subsequent detection measures and quantitative real-time PCR or isothermal target amplification. Preferably, the quantification steps comprise quantitative real-time PCR or quantitative real-time isothermal amplification. More preferably, quantification comprises quantitative real-time PCR.

Amplification methods used for quantification of nucleic acid other than PCR-based methods include but are not limited to ligase chain reaction (LCR), transcription-based amplification system (TAS), nucleic acid sequence based amplification (NASBA), rolling circle amplification (RCA), transcription-mediated amplification (TMA), self -sustaining sequence replication (3SR), Qj3 amplification and (thermostable) helicase dependent amplification ((t)HDA). NASBA, TAS, RCA, TMA, 3SR, (t)HDA and Qβ amplification are isothermal amplification methods.

In some embodiments of the invention the method additionally comprises subsequent to the ligation step a step of contacting the RNA in the sample with a reverse transcriptase under conditions allowing for the reverse transcription of RNA and/or the specific class of RNA in the sample.

Preferably, the quantification steps comprise quantitative real-time reverse transcription PCR or quantitative real-time reverse isothermal amplification.

Most preferably, the quantification steps comprise quantitative real-time reverse transcription PCR. In preferred embodiments of the invention, the quantification step(s) comprise(s) (i) me reverse transcription of RNA (*e.g*. mRNA) into DNA (*e.g.* cDNA) using a RNA-dependent DNA polymerase (*i.e*. a reverse transcriptase), (ii) the amplification of the DNA produced by reverse transcription using PCR, and (in) the detection and quantification of the amplification products in real time.

In particular embodiments reverse transcribing and quantifying are performed in the same reaction container.

In some embodiments the reverse transcriptase is a polymerase also used for amplification during the quantification steps.

For the embodiments of the present invention selective primers or random primers can be used in quantitative real-time PCR or isothermal amplification.

A "primer" herein refers to an oligonucleotide comprising a sequence that is complementary to a nucleic acid to be transcribed ("template"). During replication polymerases attach nucleotides to the 3' end of the primer complementary to the respective nucleotides of the template.

In some methods according to the present invention the two terminal regions to be ligated are part of separate nucleic acid probes. In other embodiments, the two terminal regions to be ligated are on the same nucleic acid probe.

The first nucleic acid probe in some embodiments is DNA and the ligase is a DNA ligase selected from the group consisting of T4 DNA ligase, T7 DNA ligase and *E. coli* ligase. The preferred ligase is T4 DNA Ligase.

In preferred embodiments of the invention the first nucleic acid probe is RNA and the ligase is T4 RNA ligase.

Preferably the quantity of the nucleic acid to be quantified and the total quantity of nucleic acid or the total quantity of the specific class of nucleic acid are determined at the same time.

In particular embodiments of the invention the polymerase used for quantitative real-time PCR is a polymerase from a thermophile organism or a thermostable polymerase or is selected from the group consisting of *Thermus thermophilus* (Tth) DNA polymerase, *Thermus acquaticus* (Taq) DNA polymerase, *Thermotoga maritima* (Tma) DNA polymerase, *Thermococcus litoralis* (Tli) DNA polymerase, *Pyrococcus furiosus* (Pfu) DNA polymerase, *Pyrococcus woesei* (Pwo) DNA polymerase, *Pyrococcus kodakaraensis* KOD DNA polymerase, *Thermus filiformis* (Tfi) DNA polymerase, *Sulfolobus solfataricus* Dpo4 DNA polymerase, *Thermus pacificus* (Tpac) DNA polymerase, *Thermus ggertssonii* (Teg) DNA polymerase, *Thermus brockianus* (Tbr) and *Thermus flavus* (Tfl) DNA polymerase.

In preferred embodiments of the invention the second and third nucleic acid probe are labelled with one or more fluorescent dye(s) and/or quencher(s) and wherein the quantifying steps comprise detecting fluorescence signals in the sample.

More preferably, the second and third nucleic acid probes are fluorescently labelled probes selected from the group consisting of hybridization probe, hydrolysis probe and hairpin probe.

Particularly, the fluorescently labelled probes are labelled with a dye selected from the group consisting of FAM, VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodan1Îl1e Red, Texas Red, Yakima Yellow, Alexa Fluor and PET or analogous dyes with similar excitation and emission properties.

In particular, the hybridization probe is a LightCycler probe (Roche) or the hydrolysis probe is a TaqMan probe (Roche). In other embodiments the hairpin probe is selected from the group consisting of molecular beacon, Scorpion primer, Sunrise primer, LUX primer and Amplifluor primer.

In some embodiments, the means and methods according to the present invention are used for the normalization of gene expression levels.

In some embodiments of the present invention additionally a pre-quantified nucleic acid is added to the sample and the quantity of said pre-quantified nucleic acid is determined in the quantifying steps. The pre-quantified nucleic acid may for example be a DNA, cDNA, ssDNA, RNA, *in vitro* transcribed RNA, RNA or synthetic RNA. Preferably, the pre-quantified nucleic acid is a synthetic RNA.

Preferably, the quantities of two or more nucleic acids to be quantified are normalized simultaneously, *i.e.* at the same time.

Thus, a preferred embodiment of the present invention relates to a method for the normalization of the quantity of a mRNA to be quantified in a sample to the total quantity of mRNA in the sample, comprising the steps of (a) providing a sample containing a mRNA to be quantified; (b) adding one or more first nucleic acid probes to the sample under conditions allowing for hybridization of at least a terminal region of said first nucleic acid probe to a poly-A sequence of mRNA in the sample such that double-stranded nucleic acids are created, wherein the first nucleic acid probe comprises one or more primer binding sites and optionally a probe binding site; (c) contacting the hybridized nucleic acids in the sample with a ligase under conditions that allow for ligation between any two terminal regions of such nucleic acid probe(s) whose 3' and 5' ends after hybridization are positioned in a way that ligation may occur; (d) contacting mRNA in the sample with a reverse transcriptase under conditions allowing for the reverse transcription of mRNA in me sample; (e) quantifying the total amount by quantification of the ligation product from step (c) using primers and optionally a second probe which is substantially complementary to a defined region of said first nucleic acid probe; (f) quantifying the RNA to be quantified using a third probe substantially complementary to a region of said mRNA to be quantified; and (g) normalizing the quantity of the mRNA to be quantified by determining the ratio of the quantity of the mRNA to be quantified to the total quantity of mRNA.

The present invention also relates to a kit for performing any of the above described methods, wherein the kit comprises: (a) one or more first nucleic acid probes substantially complementary to a defined region of a nucleic acid or a specific class of nucleic acids in the sample, wherein the first nucleic acid probe comprises one or more primer binding sites and optionally a probe binding site; (b) a ligase; and (c) a second nucleic acid probe substantially complementary to said probe binding site on said first nucleic acid probe.

Thus, the present invention relates to a kit for the normalized quantification of nucleic acids in a sample, wherein the kit comprises: (a) one or more first nucleic acid probes substantially complementary to defined regions of nucleic acid or a specific class of nucleic acids in the sample, wherein the first nucleic acid probe comprises one or more primer binding sites and optionally a probe binding site; (b) a ligase; and (c) a second nucleic acid probe substantially complementary to said probe binding site on said first nucleic acid probe.

In preferred embodiments of the invention, the kit additionally comprises a polymerase. The kit may additionally also comprise a nucleotide mixture and (a) reaction buffer(s) and/or a set of primers and optionally a second probe for the amplification and detection of the ligation product. In some embodiments, the kit additionally comprises a reverse transcriptase.

Thus, a preferred embodiment of the invention relates to a kit for the normalization of the quantity of a mRNA to be quantified in a sample to the total quantity of mRNA in the sample, wherein the kit comprises one or more first nucleic acid probes substantially complementary to poly-A sequences of RNA in the sample, wherein the first nucleic acid probe comprises one or more primer binding sites and optionally a probe binding site.

In particular embodiments, the kit additionally comprises one or more pre-quantified calibrator nucleic acids, a set of primers for the amplification of said calibrator nucleic acids and a first nucleic acid probe substantially complementary to a sequence on said pre-quantified nucleic acid. In some embodiments, one ore more of the components are premixed in the same reaction container.

As indicated herein above, for the analysis of gene expression, the quantification of mRNA in samples can be performed using quantitative real-time reverse transcription PCR (RT-qPCR). RT-qPCR methods employ a combination of three steps: (i) the reverse transcription of the mRNA into cDNA using a RNA-dependent DNA polymerase (*i.e.* a reverse transcriptase), (ii) the amplification of cDNA using PCR, and (iii) the detection and quantification of the amplification products in real time. For reverse transcription and PCR-based amplification, dNTPs ("nucleotide mixture") need to be present in the reaction buffer. A nucleotide mixture according to the present invention is a mixture of dNTPs, *i.e.* a mixture of dATP, dCTP, dGTP and dTTP/dUTP suitable for the use in PCR. For particular embodiments of the present invention the relative amounts of these dNTPs may be adapted according to the particular nucleotide content of the template nucleic acids. The RT-qPCR steps can either be performed in a single-stage process or in a two-stage process. In the first case, reverse transcription and PCR-based amplification are performed in the same reaction container, *e.g.* by utilizing a DNA polymerase which has intrinsic reverse transcription functionality, like *Thermus thermophilus* (Tth) polymerase or using a mixture of a reverse transcriptase and a thermostable DNA polymerase. In a two-stage setup the steps of reverse transcribing the RNA and amplifying the DNA are performed separately, *e.g.* in different reaction containers. The steps of the methods according to the present invention may be conducted in suitable reaction buffers, *e.g*. comprising salts such as magnesium ions. As already stated, the different steps may or may not be conducted in the same buffers and reaction containers. In contrast to RT-qPCR, in qPCR no reverse transcription is performed, therefore it is a quantification method for DNA rather than for RNA.

The reverse transcription of (m)RNA in RT-qPCR and the amplification of (c)DNA in qPCR and RT-qPCR need to be primed by oligonucleotides ("primers"), In the case of mRNA quantification with RT-qPCR, mRNA specific oligonucleotides can be used, *e.g.* oligo-dT primers that hybridize to the poly-A-tail of mRNA. However, also random primers of varying lengths can be utilized.

In yet another embodiment, isothermal real-time amplification reactions may be performed. Alternative to PCR for the analysis of gene expression, the quantification of mRNA in samples can be performed using quantitative real-time reverse transcription isothermal amplification, *e.g.* employing helicase-dependent amplification. Such methods employ the following steps: (i) the reverse transcription of the mRNA into cDNA using a RNA-dependent DNA polymerase (*i.e.* a reverse transcriptase), (ii) the amplification of cDNA by isothermal amplification, and (iii) the detection and quantification of the amplification products in real time. For reverse transcription and isothermal-based amplification, dNTPs ("nucleotide mixture") need to be present in the reaction buffer. A nucleotide mixture according to the present invention is a mixture of dNTPs, *i.e.* a mixture of dATP, dCTP, dGTP and dTTP/dUTP suitable for the use in PCR. For particular embodiments of the present invention the relative amounts of these dNTPs may be adapted according to the particular nucleotide content of the template nucleic acids. The RT-isothermal amplification steps can either be performed in a single-stage process or in a two-stage process. In the first case, reverse transcription and isothermal amplification are performed in the same reaction container, *e.g*. by utilizing a helicase for strand separation, a DNA polymerase which has intrinsic reverse transcription functionality, or using a mixture of a reverse transcriptase and a DNA polymerase. In a two-stage setup the steps of reverse transcribing the RNA and amplifying the DNA are performed separately, *e.g*. in different reaction containers. The steps of the methods according to the present invention may be conducted in suitable reaction buffers, *e.g*. comprising salts such as magnesium ions and may contain additional co-factors such as ATP or dATP or single-strand binding proteins. As already stated, the different steps may or may not be conducted in the same buffers and reaction containers.

Moreover, isothermal amplification protocols or standard quantitative real-time PCR protocols and kits can be adapted or amended for the means and methods according to the present invention.

As mentioned above, real-time PCR (also designated herein as quantitative PCR or quantitative real-time PCR (qPCR)) is a method to simultaneously amplify and quantify nucleic acids using a polymerase chain reaction (PCR). Quantitative real-time reverse transcription PCR (RT-qPCR) is a quantitative real-time PCR method further comprising a reverse transcription of RNA into DNA, *e.g*. mRNA into cDNA. In qPCR and RT-qPCR methods, the amplified nucleic acid is quantified as it accumulates. Typically, fluorescent dyes that intercalate with double-stranded DNA (dsDNA), *e.g*. ethidiumbromide or SYBR® Green I, or modified nucleic acid probes ("reporter probes") that fluoresce when hybridized with a complementary nucleic acid (*e.g*. the accumulating DNA) are used for quantification in qPCR based methods. Particularly, fluorogenic primers, hybridization probes (*e.g*. LightCycler probes (Roche)), hydrolysis probes (*e.g*. TaqMan probes (Roche)), or hairpin probes, such as molecular beacons, Scorpion primers (DxS), Sunrise primers (Oncor), LUX primers (Invitrogen), Amplifluor primers (Intergen) or the like can be used as reporter probes. In accordance with the present invention, fluorogenic primers or probes may for example be primers or probes to which fluorescence dyes have been attached, *e.g*. covalently attached. Such fluorescence dyes may for example be FAM (5-or 6-carboxyfluorescem), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor, PET Biosearch Blue™, Marina Blue®, Bothell Blue®, CAL Fluor® Gold, CAL Fluor® Red 610, Quasar™ 670, LightCycler Red640®, Quasar™ 705, LightCycler Red705® and the like. Particular reporter probes may additionally comprise fluorescence quenchers.

The present invention also relates to the use of the methods of the invention or the kit of the invention for the normalization of the quantity of a specific nucleic acid to the quantity of a reference nucleic acid.

Furthermore, the present invention also relates to the use of the methods of the invention or the kit of the invention gene expression analysis.

### Description of drawings

Figure 1 illustrates a particular embodiment of the invention. An open circle probe (OCP) is hybridized to a poly-A tail of RNA in a way that ligation through a ligase may occur so that the OCP is circularized. The OCP comprises two primer binding sites and a TaqMan probe binding site.
Figure 2 illustrates another embodiment of the invention where two probes hybridize to poly-A-tail of the target mRNA in a way that ligation through a ligase may occur. Each of the two probes has a dedicated primer binding site, one probe has a probe binding site, *e.g*. for a TaqMan probe.

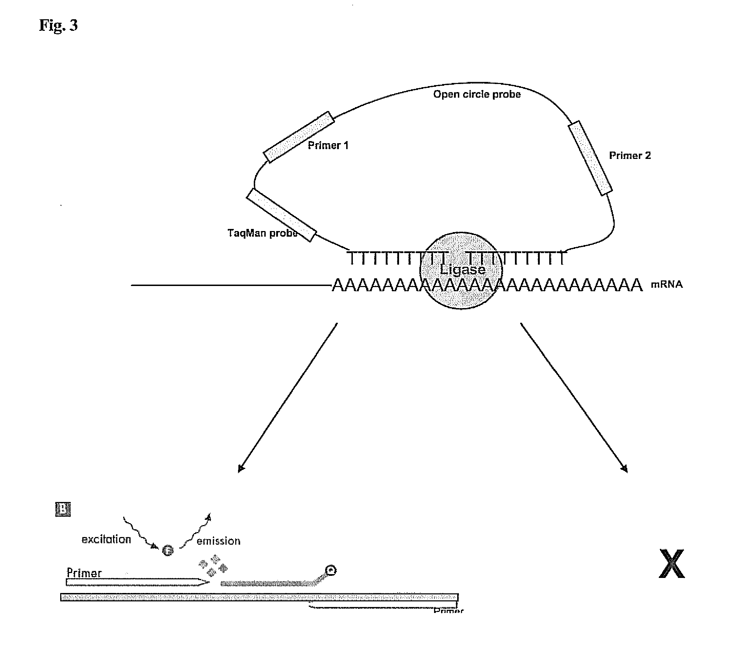

Figure 3: The principle of OCP amplification. Left arrow: In the presence of ligation (target sequence is contained in sample), an amplification product is generated by primers 1 and 2 and a detectable signal is generated, *e.g*. by probe hydrolysis. Right arrow: In the absence of ligation (target sequence not contained in sample), no amplification product is formed through primers 1 and 2 and thus no detectable signal is generated.
Figure 4: Open circle probe as illustrated in Figure 1 and used in example 1. Primer binding sites are indicated by solid and dotted boxes, respectively. A probe binding site is indicated by a dashed box.

### Examples

### Example 1: Circularization of an oligonucleotide probe on mRNA

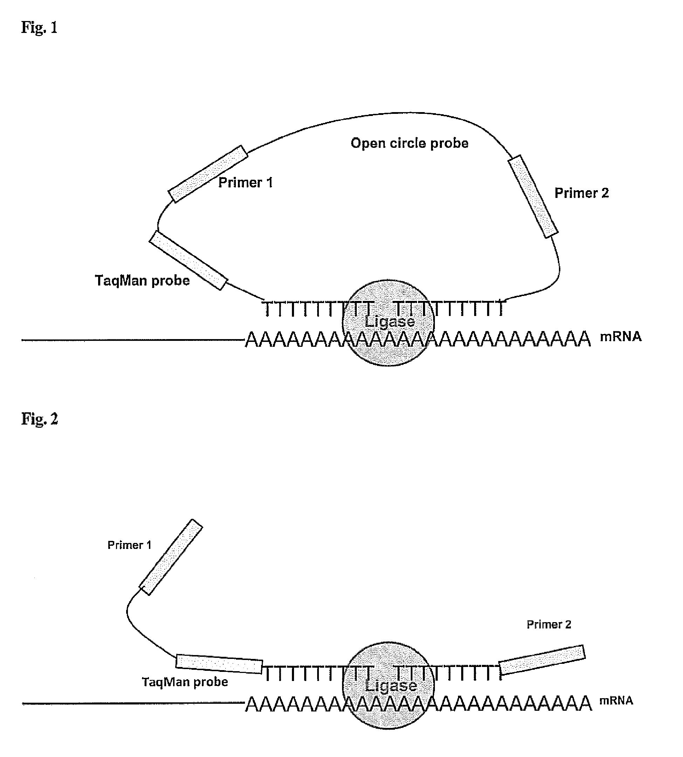

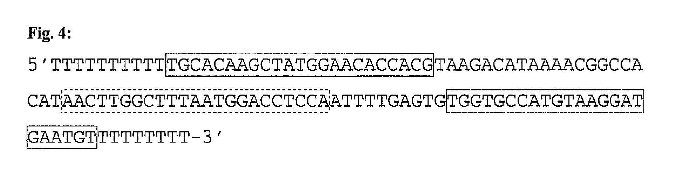

To generate an amplification target that is directly proportional to the initial amount of messenger RNA contained in the sample, a probe molecule as shown in Figure 1 having the sequence shown in Figure 4 is provided.

The poly(T) sequence at the 5'- and 3'-end of the oligonucleotide hybridize to the poly(A) portion of the mRNA. If those sequences are hybridizing in a head-to-tail configuration on the mRNA target template, the ligase is able join the ends creating a covalently closed single-stranded target nucleic acid that can be amplified with primers specific to this circularized DNA.

Circularization of the probe on a mRNA target was conducted according to the following experimental conditions: Either 80 ng, 40 ng or 20 ng leukocyte RNA was mixed each with 1 µl T4 Ligase, 4 µl T4-Ligase ligation buffer, 6µl water and incubated for 60 min at room temperature in a final reaction volume of 20 µl. A negative control was included that did not comprise target RNA. Each reaction also contained either 0.1 µM or 0.01 µM of the oligonucleotide shown in Figure 4.

### Example 2: Amplification of circularized target oligonucleotides as an indication of mRNA content of a sample

Amplification of the circularized target oligonucleotides was conducted using the ligation reactions described in Example 1. The ligation reaction was either diluted 1:10 or 1:100 and 2 µl of those dilutions were subjected to an amplification reaction containing in a final volume of 20 µl: 2x QuantiFast SYBR Green PCR Kit Master Mix (QIAGEN), and primers at a concentration of 1 µM, The primer sequences were as follows: Primer 1: 5'-tggaggtccattaaagccaagtt-3' and Primer 2: 5'-tggtgccatgtaaggatgaatgt-3'. Following a hot start polymerase reactivation step at 95°C for 5 min, the cycling protocol consisted of 40 cycles at 95°C for 10 sec and 60°C for 30 sec. Signal was detected using real-time monitoring of the reaction using SYBR Green I dsDNA specific binding dye on an ABI PRISM 7500 Sequence Detection Instrument. Alternatively, a sequence specific fluorescent labelled probe complementary to the blue coloured sequence in Figure 1 can be also used for detection. The data shown in Table 1 clearly show a threshold cycle (Ct) value proportional to the original RNA input amount (2fold template dilution shall yield an approximately shift of one Ct) demonstrating that a circularisation and detection of a single-.stranded oligonucleotide hybridizing to the poly(A) portion of a messenger RNA molecule can be employed to detect the amount of mRNA contained in a sample and can thus be employed as a tool to normalize overall gene expression levels to the total mRNA content of a sample. In the presence of no target RNA (NTC), no signal is detected. All amplification reactions have been performed thrice and the mean Ct value has been calculated.

**Table 1: Experimental Ct values for different RNA concentrations**

| RNA (ng) in 20 µl ligation | Ct | Mean Ct |
|---|---|---|
| 80 | 15.86 | 15.88 |
| | 15.83 | |
| | 15.95 | |
| 40 | 16.96 | 16.94 |
| | 16.98 | |
| | 16.87 | |
| 20 | 18.48 | 18.73 |
| | 18.72 | |
| | 19.00 | |
| NTC | Undetermined | |
| | Undetermined | |
| | Undetermined | |

| | | |
|---|---|---|
| *NTC: Template-free control reactions* | | |

## Claims

1. A method for the normalization of the quantity of a nucleic acid to be quantified in a sample to
(i) the total quantity of nucleic acid in the sample; or to
(ii) the total quantity of a specific class of nucleic acid in the sample, comprising the steps of
(a) providing a sample containing a nucleic acid to be quantified;
(b) adding one or more first nucleic acid probes to the sample under conditions allowing for hybridization of at least a terminal region of said first nucleic acid probe(s) to a specific binding site of nucleic acid in the sample such that double-stranded nucleic acids are created, wherein the first nucleic acid probe(s) comprises one or more primer binding sites and optionally a probe binding site;
(c) contacting the hybridized nucleic acids in the sample with a ligase under conditions that allow for ligation between any two terminal regions of such nucleic acid probe(s) whose 3' and 5' ends after hybridization are positioned in a way that ligation may occur;
(d) quantifying the total amount of nucleic acid or the total amount of the specific class of nucleic acid by quantification of the ligation product from step (c) using a second probe which is substantially complementary to a defined region of said first nucleic acid probe(s) or an intercalating dsDNA specific fluorescent dye;
(e) quantifying the nucleic acid to be quantified using a third probe substantially complementary to a region of said nucleic acid to be quantified or an intercalating dsDNA specific fluorescent dye; and
(f) normalizing the quantity of the nucleic acid to be quantified by determining the ratio of the quantity of the nucleic acid to be quantified to the total quantity of nucleic acid or the total quantity of the specific class of nucleic acid.

2. The method according to claim 1, wherein the nucleic acid to be quantified is a RNA and the specific class of nucleic acid is RNA.

3. The method according to claim 2, wherein the RNA to be quantified is RNA selected from the group consisting of mRNA, rRNA, tRNA, mRNA, siRNA, snRNA, snoRNA, scaRNA, microRNA, dsRNA, ribozyme, riboswitch and viral RNA and wherein the total quantity of RNA is selected from the group consisting of the total quantity of RNA, the total quantity of mRNA, the total quantity of rRNA, the total quantity of tRNA, the total quantity of nRNA, the total quantity of siRNA, the total quantity of snRNA, the total quantity of snoRNA, the total quantity of scaRNA, the total quantity of microRNA, the total quantity of dsRNA, the total quantity of ribozyme, the total quantity of riboswitch, the total quantity of viral RNA.

4. The method according to claim 3, wherein the RNA to be quantified is a mRNA and the specific class of nucleic acid is mRNA.

5. The method according to claim 4, wherein the specific binding site is specific for mRNA, is a homopolymeric sequence or is a poly-A sequence or a poly-A tail.

6. The method according to claim 1, wherein the nucleic acid to be quantified is a DNA and the specific class of nucleic acid is DNA.

7. The method according to claim 6, wherein the DNA to be quantified is DNA selected from the group consisting of cDNA, dsDNA, ssDNA, plasmid DNA, cosmid DNA, chromosomal DNA, viral DNA and mtDNA and wherein the total quantity of DNA is selected from the group consisting of the total quantity of DNA, the total quantity of cDNA, the total quantity of dsDNA, the total quantity of ssDNA, the total quantity of plasmid DNA, the total quantity of cosmid DNA, the total quantity of chromosomal DNA, the total quantity of viral DNA and the total quantity of mtDNA.

8. The method according to claim 7, wherein the DNA to be quantified is a cDNA and the specific class of nucleic acid is cDNA.

9. The method according to claim 8, wherein the specific binding site is a poly-T sequence or a poly-T tail.

10. The method according to any one of the claims 1 to 9, wherein quantification comprises quantitative real-time PCR or quantitative real-time isothermal amplification.

11. The method according to any one of the claims 2 to 5, wherein subsequent to the ligation step the method additionally comprises a step of contacting the RNA in the sample with a reverse transcriptase under conditions allowing for the reverse transcription of RNA and/or the specific class of RNA in the sample.

12. The method according to any one of the claims 1 to 11, wherein the two terminal regions to be ligated are part of separate nucleic acid probes or are on the same nucleic acid probe.

13. The method according to any one of the claims 1 to 12, wherein the second and third nucleic acid probe are labelled with one or more fluorescent dye(s) and wherein the quantifying steps comprise detecting fluorescence signals in the sample.

14. A kit for performing the method of any one of the claims 1 to 13, wherein the kit comprises:
(a) one or more first nucleic acid probes substantially complementary to a defined region of a nucleic acid or a specific class of nucleic acids in the sample, wherein the first nucleic acid probe comprises one or more primer binding sites and optionally a probe binding site;
(b) a ligase; and
(c) a second nucleic acid probe substantially complementary to said probe binding site on said first nucleic acid probe or a dsDNA specific fluorescent dye.

15. The use of the methods according to any one of the claims 1 to 13 or the kit according to claim 14 for the normalization of the quantity of a specific nucleic acid to the quantity of a reference nucleic acid or for gene expression analysis.
